# EUROPÄISCHE PATENTSCHRIFT

(11) **EP 2 361 544 B1**
(45) Veröffentlichungstag und Bekanntmachung des Hinweises auf die Patenterteilung: **03.06.2015**
(21) Anmeldenummer: 11154479.7
(22) Anmeldetag: 15.02.2011
(51) Int. Cl.: A61B 1/00, A61B 1/005

(54) **Endoskop**
Endoscope
Endoscope

(30) Priorität: 25.02.2010 DE 102010002334
(43) Veröffentlichungstag der Anmeldung: 31.08.2011
(73) Patentinhaber: Henke-Sass, Wolf GmbH, 78532 Tuttlingen (DE)
(72) Erfinder: Häckl, Norbert, 88637, Leibertingen (DE); Seeh, Daniel, 78194, Immendingen (DE)
(74) Vertreter: Geyer, Fehners & Partner

(56) Entgegenhaltungen:
- JP-A- 2009 045 134
- US-A- 4 867 136
- US-A- 5 536 235

## Beschreibung

Die vorliegende Erfindung bezieht sich auf ein Endoskop gemäß dem Oberbegriff des Anspruches 1, wie es z.B. aus der JP 2009-045134 A bekannt ist

Bisher war es bei Endoskopen üblich, das distale Ende des Außenschlauchs mit einem Garn oder Faden zu umwickeln, um es dadurch auf der Auflage zu fixieren.

Es hat sich jedoch gezeigt, daß eine solche Art der Fixierung nicht dauerhaft ist. Insbesondere hält diese Art der Fixierung nur einer begrenzten Anzahl von Autoklavierungszyklen stand.

Aus der US 4,867,136 ist ein Endoskop bekannt, das einen Handgriff und ein mit dem Handgriff verbundenen Endoskopschaft, der einen abwinkelbaren Abschnitt und einen sich daran anschließenden starren Abschnitt mit einer sich in Längsrichtung des Endoskopschafts erstreckenden Auflage aufweist, bekannt, wobei der abwinkelbare Abschnitt einen flexiblen Außenschlauch aufweist, dessen dem starren Abschnitt zugewandtes Ende auf der Auflage aufliegt, wobei der flexible Außenschlauch mit einem Garn oder Faden umwickelt ist, um ihn dadurch auf der Auflage zu fixieren.

Aus der US 5,536,235 ist ein Endoskop bekannt, bei dem ein flexibler Außenschlauch, der auf einer.Auflage eines Handgriffs des Endoskops aufliegt, mit einer übergreifenden Hülse fixiert ist.

Ausgehend hiervon ist es daher Aufgabe der Erfindung, ein Endoskop der eingangs genannten Art so zu verbessern, daß die Fixierung des distalen Endes des Außenschlauchs auf der Auflage dauerhaft gewährleistet werden kann, selbst wenn das Endoskop häufig autoklaviert wird.

Die Aufgabe wird bei einem Endoskop der eingangs genannten Art dadurch gelöst, daß die Fixierhülse einen Fixierbereich mit sich verjüngendem Innendurchmesser aufweist, die Auflage einen ersten Bereich mit, in Richtung vom abwinkelbaren Abschnitt weg, zunehmendem Außendurchmesser aufweist und die Auflage in Richtung vom abwinkelbaren Abschnitt weg einen an den ersten Bereich anschließenden zweiten Bereich mit abnehmendem Außendurchmesser aufweist.

Da die Fixierhülse den Fixierbereich mit sich verjüngendem Innendurchmesser aufweist, verringert sich der Abstand zwischen der Fixierhülse und der Auflage in axialer Richtung, wodurch in axialer Richtung die Klemmkraft, die auf das distale Ende des Außenschlauches wirkt, zunimmt. Dies führt zu einer besseren Fixierung.

Die Auflage weist den ersten Bereich mit, in Richtung vom abwinkelbaren Abschnitt weg, zunehmenden Außendurchmesser auf. Dabei kann der Fixierbereich der Fixierhülse dem ersten Bereich der ersten Auflage gegenüberliegen. Dies führt zu einer weiteren Verringerung des Abstandes zwischen Fixierhülse und Auflage, so daß eine weitere Erhöhung der Klemmkfäft erreicht wird.

Des weiteren weist die Auflage in Richtung vom abwinkeibaren Abschnitt weg den an den ersten Bereich anschließenden zweiten Bereich mit abnehmendem Außendurchmesser auf. Damit nimmt zwar der Abstand zwischen der Auflage (im Bereich des zweiten Bereiches) und der Fixierhülse wieder zu. Insgesamt führt dies aber zu einer Verbesserung der Beständigkeit der Klemmung hinsichtlich axial an den Außenschlauch angreifenden Zugkräften.

Da die Fixierhülse mit dem starren Abschnitt verbunden ist, die das dem starren Abschnitt zugewandte Ende des Außenschlauchs übergreift, das zur Fixierung zwischen der Fixierhülse und der Auflage eingeklemmt ist, wird somit eine Klemmung des dem starren Abschnitt zugewandten Endes des Außenschlauches zwischen der Fixierhülse und der Auflage erreicht, wodurch eine dauerhafte Fixierung gewährleistet werden kann. Insbesondere hält diese Art der Klemmung auch einer Vielzahl von Autoklavierungszyklen stand. Unter Autöklavieren wird hier insbesondere verstanden, daß das Endoskop bzw. der Endoskopschaft mindestens mehrere Minuten gesättigtem Wasserdampf von ca. 120°C - 140°C zur Sterilisation ausgesetzt wird. Damit kann das Endoskop sterilisiert werden, was insbesondere für medizinische Anwendungen wichtig ist.

Die Fixierhülse kann auf dem starren Abschnitt in Längsrichtung des Endoskopschaftes verschraubbar gelagert sein. Somit ist es möglich, die Fixierhülse auf den Endabschnitt aufzuschrauben und dadurch die gewünschte Klemmung zu realisieren.

Es ist natürlich auch jede andere Art der Verbindung zwischen der Fixierhülse und dem starren Abschnitt möglich. Insbesondere kann die Fixierhülse mit dem starren Abschnitt verklebt, verlötet, etc. sein.

Bevorzugt kann am starren Abschnitt des Schaftes eine Aufnahmehülse befestigt sein, die einen die Auflage bildenden Auflagebereich aufweist. Dadurch kann die Herstellung des Endoskops vereinfacht werden. Insbesondere kann die Aufnahmehülse an die speziellen Anforderungen des jeweiligen Endoskops leichter angepaßt werden.

Ferner kann die Aufnahmehülse einen Befestigungsbereich aufweisen, mit dem die Fixierhülse direkt verbunden ist. Bei dem Befestigungsbereich kann es sich insbesondere um ein Außengewinde handeln, so daß die Fixierhülse auf dem Befestigungsbereich aufgeschraubt werden kann.

Bei dem erfindungsgemäßen Endoskop kann das dem starren Abschnitt zugewandte Ende des Außenschlauches mit einem Faden oder Garn umwinkelt sein, der das dem starren Abschnitt zugewandte Ende auf die Auflage drückt. Ferner kann der Garn oder Faden zusätzlich mit einem Klebemittel, wie z.B. einem Harz, getränkt oder beaufschlagt sein. Damit wird eine weitere Erhöhung der Fixierung erreicht.

Unter dem starren Abschnitt des Endoskopschaftes wird hier ein Abschnitt verstanden, der im Gegensatz zu dem abwinkelbaren Abschnitt nicht abgewinkelt werden kann. Natürlich weist der starre bzw. steife Abschnitt eine gewisse Elastizität auf, so daß eine gewisse Biegung möglich ist. Er ist aber nicht in der Art abwinkelbar, wie der abwinkelbare Abschnitt, der über Betätigungselemente am Handgriff reproduzierbar abgewinkelt werden kann.

Bei dem starren Abschnitt kann es sich um einen solchen Abschnitt des Endoskopschaftes handeln, der mit dem distalen oder proximalen Ende des abwinkelbaren Abschnittes verbunden ist. Insbesondere kann es sich bei dem starren Abschnitt um den distalen Endabschnitt des Endoskopschaftes handeln. Dabei kann der distale Endabschnitt z.B. eine Abbildungsoptik für die Abbildung der gewünschten Bereiche enthalten. Auch kann im distalen Endabschnitt ein Bildsensor enthalten sein, auf den die Abbildungsoptik das Bild abbildet. Alternativ ist es möglich, daß der Abbildungsoptik eine Übertragungsoptik nachgeordnet ist, die durch den abwinkelbaren Abschnitt bis zum Handgriff des Endoskops verläuft.

Das erfindungsgemäße Endoskop oder zumindest der Endoskopschaft des erfindungsgemäßen Endoskops ist hermetisch dicht ausgebildet und kann daher autoklaviert werden.

Ferner kann bei dem erfindungsgemäßen Endoskop der Endoskopschaft zwischen dem Handgriff und dem abwinkelbaren Abschnitt einen starren Hauptteil aufweisen, der mit dem abwinkelbaren Abschnitt verbunden ist, wobei der Hauptteil eine sich in Längsrichtung des Endoskopschaftes erstreckende zweite Auflage aufweist, auf der das proximale Ende des flexiblen Außenschlauches aufliegt, wobei eine zweite Fixierhülse mit dem Hauptteil verbunden ist und das proximale Ende des Außenschlauches übergreift, das zur Fixierung zwischen der zweiten Fixierhülse und der zweiten Auflage eingeklemmt ist.

Damit kann somit auch das andere Ende (das proximale Ende, das zum Handgriff des Endoskops hinweist) des Außenschlauches gut und dauerhaft am Hauptteil fixiert werden.

Die zweite Fixierhülse sowie die zweite Auflage können in gleicher Weise gebildet werden wie die Fixierhülse und die Auflage zur Fixierung des distalen Endes des Außenschlauches.

Das erfindungsgemäße Endoskop kann noch weitere dem Fachmann bekannte Elemente bzw. Bauteile enthalten, die zum Betrieb des Endoskops notwendig sind. Insbesondere können im und am Handgriff weitere Bauelemente vorgesehen bzw. angeschlossen werden.

Bei dem erfindungsgemäßen Endoskop handelt es sich bevorzugt um ein medizinisches Endoskop.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Seitenansicht einer Ausführungsform des erfindungsgemäßen Endoskops;
- Fig. 2: eine Seitenansicht des Endoskops von Fig. 1 mit nach unten geschwenktem distalen Endabschnitt des Endoskopschaftes;
- Fig. 3: eine vergrößerte Schnittdarstellung des Details A von Fig. 1, und
- Fig. 4: eine vergrößerte Ansicht des Details B von Fig. 3.

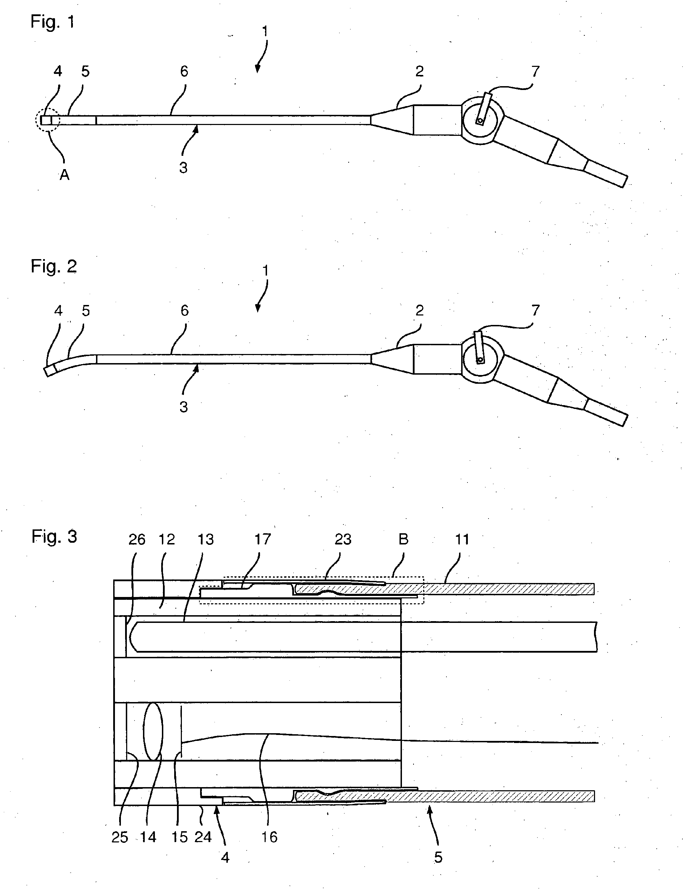

Bei der in Fig. 1 bis 4 gezeigten Ausführungsform umfaßt das erfindungsgemäße Endoskop 1 einen Handgriff 2 und ein mit dem Handgriff 2 verbundener Schaft 3.

Der Schaft 3 ist fast über seine gesamte Länge als starrer Schaft 3 ausgebildet, wobei ein starrer distaler Endabschnitt 4 über einen abwinkelbaren Abschnitt 5 mit einem starren Hauptteil 6 des Schaftes 3 gelenkig verbunden ist, so daß der distale Endabschnitt 4 relativ zum restlichen Teil 6 verschwenkbar ist. Wie in Fig. 2 angedeutet ist, kann z.B. der distale Endabschnitt 4 nach unten abgelenkt werden, indem ein Steuerhebel 7 am Handgriff 2 von der in Fig. 1 gezeigten Position in die in Fig. 2 gezeigte Position geschwenkt wird.

Der abwinkelbare Abschnitt 5 weist mehrere miteinander gelenkig verbundene Rohrsegmente, die zur Vereinfachung der Darstellung nicht gezeigt sind, auf, um die gewünschte Biegbarkeit zu erreichen. Die genaue Ausbildung solcher Rohrsegmente ist dem Fachmann bekannt und soll nicht detailliert beschrieben werden. Als Beispiel für eine mögliche Ausbildung der Rohrsegmente wird auf die DE 10 2004 027 850 A1 verwiesen, deren Inhalt hiermit in die vorliegende Beschreibung aufgenommen wird. Die Rohrsegmente können z.B. gemäß Figuren 11A - 11C der DE 10 2004 027 850 A1 ausgebildet sein.

Auch die Betätigung des abwinkelbaren Abschnitts 5 kann in gleicher Weise wie in der DE 10 2004 027 850 A1 über die Steuerzüge (nicht gezeigt) erfolgen, wobei hier die Steuerzüge mit dem Steuerhebel 7 verbunden sind. Somit kann über den Steuerhebel 7 der Endabschnitt 4 nach unten (wie in Fig. 2 gezeigt ist) und nach oben (nicht gezeigt) verschwenkt werden. Der abwinkelbare Abschnitt 5 kann hier insbesondere so ausgebildet sein, daß ein Verschwenken des Endabschnittes 4 senkrecht zur Zeichenebene gemäß Fig. 1 und 2 ebenso möglich ist. Dazu ist dann ein zweiter Steuerhebel (nicht gezeigt) am Handgriff 2 vorgesehen, um diese Schwenkbewegung durchzuführen.

Der abwinkelbare Abschnitt 5 weist ferner einen flexiblen Außenschlauch 11 auf, der mit dem distalen Endabschnitt 4 so verbunden ist, daß eine hermetische dichte Umhüllung für die Rohrsegmente und alle darin enthaltene Elemente bereitgestellt ist.

Wie der vergrößerten Schnittdarstellung des Details A von Fig. 1 in Fig. 3 zu entnehmen ist, weist der distale Endabschnitt 4 einen Hauptkörper 12 auf, in dem ein durch den abwinkelbaren Abschnitt 5 laufendes Lichtleiterbündel 13 zur Beleuchtung endet und in dem eine Aufnahmeoptik 14 zur Aufnahme der gewünschten Bilder angeordnet ist. Die Aufnahmeoptik 14 ist hier schematisch als Linse dargestellt, die das Bild auf einen Bildsensor 15 abbildet. Die elektrischen Bildsignale des Bildsensors 15 werden über eine durch den abwinkelbaren Abschnitt 5 und das Hauptteil 6 verlaufende Leitung 16 zum Handgriff 2 übertragen.

Alternativ ist es möglich, daß der Aufnahmeoptik 14 ein sich durch den abwinkelbaren Abschnitt 5 und den Hauptteil 6 erstreckendes Lichtleitsystem (nicht gezeigt) nachgeordnet ist, das das aufgenommene Bild bis zum Handgriff 2 überträgt.

In der Schnittdarstellung von Fig. 3 ist zur Vereinfachung der Darstellung lediglich der Außenschlauch 11 schraffiert gezeichnet.

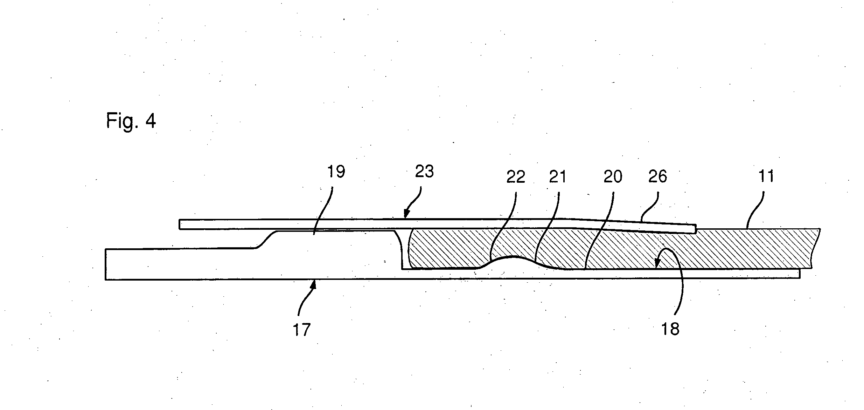

Am Hauptkörper 12 ist eine Aufnahmehülse 17 befestigt, die, wie am besten in Fig. 4 ersichtlich ist, einen Auflagebereich 18 und einen sich daran anschließenden Befestigungsbereich 19 mit einem Außengewinde aufweist. In Fig. 4 ist das Detail B von Fig. 3 vergrößert dargestellt. Der Auflagebereich 18 weist von seinem zum abwinkelbaren Abschnitt 5 weisenden Ende in Richtung zum Endabschnitt 4 zunächst einen Bereich 20 mit konstantem Außendurchmesser auf, der in einen Bereich 21 mit zunehmendem Außendurchmesser übergeht, an den sich ein Bereich 22 mit abnehmendem Außendurchmesser anschließt, der sich bis zum Befestigungsbereich 19 erstreckt. Auf den Bereichen 20 bis 22 liegt der Außenschlauch 11 auf. Auf den Befestigungsbereich 19 der Aufnahmehülse 17 ist eine Fixierhülse 23 aufgeschraubt, die sich im aufgeschraubten Zustand über die Bereiche 21 und 22 sowie teilweise über den Bereich 20 der Aufnahmehülse 17 erstreckt und dadurch das dem Endabschnitt zugewandte Ende des Außenschlauches 11 übergreift bzw. auf seiner Außenseite aufliegt und somit das dem Endabschnitt zugewandte Ende gegen die Aufnahmehülse 17 drückt. In dieser Art wird der Außenschlauch 11 zwischen Aufnahmehülse 17 und Fixierhülse 23 eingeklemmt.

Die Fixierhülse 23 ist hier so ausgebildet, daß ihr Innendurchmesser in Richtung zum abwinkelbaren Abschnitt 5 hin abnimmt, so daß auch der Abstand zwischen Fixierhülse 23 und Aufnahmehülse 17 in Richtung zum abwinkelbaren Abschnitt 5 hin geringer wird. Dies führt zu einer guten Fixierung des Außenschlauches 11 auf der Aufnahmehülse 17. Der Bereich der Fixierhülse 23, in dem sich der Innendurchmesser verringert, kann auch als Fixierbereich 26 bezeichnet werden.

Die Bereiche 21 und 22 der Aufnahmehülse 17 bilden eine lokale Erhöhung, so daß der Abstand zwischen der Fixierhülse 23 und der Aufnahmehülse 17 vor und hinter der lokalen Erhöhung (in axialer Richtung; in Fig. 4 von rechts nach links) größer ist als im Bereich der lokalen Erhöhung. Damit wird in diesem Bereich der flexible Außenschlauch 11 am stärksten zusammengedrückt, wodurch die Klemmung des Außenschlauches 11 höheren an den Außenschlauch 11 angreifenden axialen Zugkräften standhält.

Insbesondere ist die durch die Aufnahmehülse 17 und die Fixierhülse 23 bewirkte Klemmung des Außenschlauches 11 hermetisch dicht. Selbst eine Vielzahl von Autoklavierungszyklen führen zu keinen Undichtigkeiten, so daß eine ausgezeichnete Fixierung des Außenschlauches 11 am distalen Endabschnitt 4 bereitgestellt ist, die mechanisch äußerst stabil sowie dauerhaft autoklavierbar ist.

In einer Weiterbildung ist es möglich, daß vor Aufschrauben der Fixierhülse 23 der auf dem Auflagebereich 18 aufliegende distale Endabschnitt des Außenschlauches 11 mit einem Garn oder Faden (beispielsweise einem Nylongarn) umwickelt und dadurch gegen den Auflagebereich 18 gedrückt wird. Der Garn bzw. Faden kann zusätzlich mit einem Klebemittel (beispielsweise Kleber, Harz, etc.) getränkt oder beaufschlagt sein, so daß eine weitere Verbesserung der Fixierung erreicht wird. Über diesen Faden bzw. Garn liegt dann die Fixierhülse 23, was zu einer weiteren Verbesserung der Fixierung führt.

Die beschriebene Fixierung des Außenschlauches 11 kann in gleicher Weise für das proximale Ende des Außenschlauches 11 durchgeführt werden, um dieses mit dem Hauptteil 6 zu verbinden. Beispielsweise können die in Fig. 4 gezeigte Aufnahmehülse 17 sowie die Fixierhülse 23 verwendet werden. In dieser Art und Weise ist eine hermetisch dichte Verbindung des flexiblen Außenschlauches 11 sowohl mit dem distalen Endabschnitt 4 als auch mit dem Hauptteil 6 möglich.

Natürlich muß die Aufnahmehülse 17, die gemäß der Darstellung in Fig. 3 mittels einer Befestigungshülse 24 am Hauptkörper 12 befestigt ist, kein separates Bauteil sein. Es ist auch möglich, daß die Aufnahmehülse in den Hauptkörper 12 bzw. in den Hauptteil 6 einstückig integriert ist.

Der Außenschlauch 11 ist bevorzugt aus einem elastischen Polymer (z.B. ein Fluorelastomer) hergestellt. Es kann natürlich jedes andere elastische Material verwendet werden, wobei Materialien bevorzugt werden, die autoklavierbar sind.

Die starren Elemente des Schaftes 3 und insbesondere die Fixierhülse 23 sowie die Aufnahmehülse 17 können aus einem Metall oder einer Metalllegierung hergestellt sein. Bevorzugt wird Edelstahl verwendet.

Das erfindungsgemäße Endoskop 1 kann noch weitere dem Fachmann bekannte Elemente aufweisen, die zum Betrieb notwendig sind. So ist das hier in Verbindung mit Fig. 1 bis 4 beschriebene Endoskop 1 autoklavierbar. Dazu sind z.B. im Hauptkörper 12 Deckgläser 25 und 26 vor der Abbildungsoptik 14 sowie dem Lichtleiterbündel 13 angeordnet, um die gewünschte hermetische Dichtheit zu erzielen. Die Deckgläser 25, 26 sind bevorzugt aus Saphirglas hergestellt.

## Patentansprüche

1. Endoskop mit
einem Handgriff (2) und
einem mit dem Handgriff (2) verbundenen Endoskopschaft (3), der einen abwinkelbaren Abschnitt (5) und einen sich daran anschließenden starren Abschnitt (4, 6) mit einer sich in Längsrichtung des Endoskopschaftes (3) erstreckenden Auflage (18) aufweist, wobei der abwinkelbare Abschnitt (5) einen flexiblen Außenschlauch (11) aufweist, dessen dem starren Abschnitt (4, 6) zugewandtes Ende auf der Auflage (18) aufliegt,
wobei eine Fixierhülse (23) mit dem starren Abschnitt (4) verbunden ist, die das dem starren Abschnitt (4, 6) zugewandte Ende des Außenschlauches (11) so übergreift, daß es zur Fixierung zwischen der Fixierhülse (23) und der Auflage (18) eingeklemmt ist **dadurch gekennzeichnet, daß**
die Fixierhülse (23) einen Fixierbereich (26) mit sich verjüngendem Innendurchmesser aufweist, die Auflage (18) einen ersten Bereich (21) mit, in Richtung vom abwinkelbaren Abschnitt (5) weg, zunehmendem Außendurchmesser aufweist und
die Auflage (18) in Richtung vom abwinkelbaren Abschnitt (4) weg einen an den ersten Bereich (21) anschließenden zweiten Bereich (22) mit abnehmendem Außendurchmesser aufweist.

2. Endoskop nach Anspruch 1, **dadurch gekennzeichnet, daß** die Fixierhülse (23) auf dem starren Abschnitt (4, 6) in Längsrichtung des Endoskopschaftes (3) verschraubbar gelagert ist.

3. Endoskop nach Anspruch 1 oder 2, **dadurch gekennzeichnet, daß** sich der Fixierbereich (26) mit dem sich verjüngenden Innendurchmesser über den ersten und zweiten Bereich (21, 22) erstreckt.

4. Endoskop nach Anspruch 1 oder 2, **dadurch gekennzeichnet, daß** der Fixierbereich (26) der Fixierhülse (23) dem ersten Bereich (21) der ersten Auflage (18) gegenüberliegt.

5. Endoskop nach einem der obigen Ansprüche, **dadurch gekennzeichnet, daß** am starren Abschnitt (4) eine Aufnahmehülse befestigt ist, die einen die Auflage (18) bildenden Auflagebereich aufweist..

6. Endoskop nach Anspruch 5, **dadurch gekennzeichnet, daß** die Aufnahmehülse einen Befestigungsbereich (19) aufweist, mit dem die Fixierhülse (23) direkt verbunden ist.

7. Endoskop nach einem der obigen Ansprüche, **dadurch gekennzeichnet, daß** der starre Abschnitt (4) der distale Endabschnitt (4) des Endoskopschaftes (3) ist.

8. Endoskop nach Anspruch 7, **dadurch gekennzeichnet, daß** der Endoskopschaft (3) zwischen dem Handgriff (2) und dem abwinkelbaren Abschnitt (5) einen starren Hauptteil (6) aufweist, der mit dem abwinkelbaren Abschnitt (5) verbunden ist, wobei der Hauptteil (6) eine sich in Längsrichtung des Endoskopschaftes (3) erstreckende zweite Auflage aufweist, auf der das proximale Ende des flexiblen Außenschlauches (11) aufliegt, wobei eine zweite Fixierhülse mit dem Hauptteil (6) verbunden ist und das proximale Endes des Außenschlauches (11) so übergreift, daß es zur Fixierung zwischen der zweiten Fixierhülse und der zweiten Auflage eingeklemmt ist.

## Claims

1. Endoscope with
a handle (2) and
an endoscope shaft (3), connected to the handle (2), which has a bendable section (5) and a rigid section (4, 6), adjoining it, with a support (18) extending in the longitudinal direction of the endoscope shaft (3), wherein the bendable section (5) has a flexible outer tube (11) the end of which facing the rigid section (4, 6) rests on the support (18),
wherein a fixing sleeve (23) is connected to the rigid section (4) and covers the end of the outer tube (11) facing the rigid section (4, 6) such that said end is clamped for fixing between the fixing sleeve (23) and the support (18)
**characterized in that**
the fixing sleeve (23) has a fixing area (26) with a tapering internal diameter,
the support (18) has a first area (21) with an external diameter increasing in the direction away from the bendable section (5) and
the support (18) has a second area (22), adjoining the first area (21), with a decreasing external diameter in the direction away from the bendable section (4).

2. Endoscope according to claim 1, the fixing sleeve (23) is mounted, screwable, on the rigid section (4, 6) in the longitudinal direction of the endoscope shaft (3).

3. Endoscope according to claim 1 or 2, **characterized in that** the fixing area (26) with the tapering internal diameter extends over the first and second areas (21, 22).

4. Endoscope according to claims 1 or 2, **characterized in that** the fixing area (26) of the fixing sleeve (23) lies opposite the first area (21) of the first support (18).

5. Endoscope according to one of the above claims, **characterized in that** a housing sleeve which has a support area forming the support (18) is fastened to the rigid section (4).

6. Endoscope according to claim 5, **characterized in that** the housing sleeve has a fastening area (19) to which the fixing sleeve (23) is directly connected.

7. Endoscope according to one of the above claims, **characterized in that** the rigid section (4) is the distal end section (4) of the endoscope shaft (3).

8. Endoscope according to claim 7, **characterized in that**, between the handle (2) and the bendable section (5), the endoscope shaft (3) has a rigid main part (6) which is connected to the bendable section (5), wherein the main part (6) has a second support extending in the longitudinal direction of the endoscope shaft (3), on which the proximal end of the flexible outer tube (11) rests, wherein a second fixing sleeve is connected to the main part (6) and covers the proximal end of the outer tube (11), such that said proximal end is clamped for fixing between the second fixing sleeve and the second support.

## Revendications

1. Endoscope, comportant
une poignée (2) et
une tige (3), qui est reliée à la poignée (2) et comporte un tronçon pliable (5) et un tronçon rigide (4, 6), adjacent à celui-ci et comportant un support (18) s'étendant dans le sens longitudinal de la tige (3) de l'endoscope, le tronçon pliable (5) comportant un tube extérieur (11) flexible, dont l'extrémité orientée vers le tronçon rigide (4, 6) repose sur le support (18),
une gaine d'immobilisation (23) étant reliée au tronçon rigide (4), laquelle s'engage au-dessus de l'extrémité du tube extérieur (11) orientée vers le tronçon rigide (4, 6), de telle sorte que, en vue de l'immobilisation, ladite extrémité est bloquée entre la gaine d'immobilisation (23) et le support (18),
**caractérisé en ce que**
la gaine d'immobilisation (23) comporte une zone d'immobilisation (26) avec un diamètre intérieur se rétrécissant, le support (18) comporte une première zone (21) avec un diamètre extérieur qui augmente dans le sens s'écartant du tronçon pliable (5), et
le support (18), dans le sens s'écartant du tronçon pliable (4), comporte une deuxième zone (22), adjacente à la première zone (21) et ayant un diamètre extérieur décroissant.

2. Endoscope selon la revendication 1, **caractérisé en ce que** la gaine d'immobilisation (23) est montée de manière à pouvoir être vissée sur le tronçon rigide (4, 6) dans le sens longitudinal de la tige (3) de l'endoscope.

3. Endoscope selon la revendication 1 ou 2, **caractérisé en ce que** la zone d'immobilisation (26) s'étend avec son diamètre intérieur se rétrécissant au-dessus de la première et de la deuxième zone (21, 22).

4. Endoscope selon la revendication 1 ou 2, **caractérisé en ce que** la zone d'immobilisation (26) de la gaine d'immobilisation (23) est située face à la première zone (21) du premier support (18).

5. Endoscope selon l'une quelconque des revendications précédentes, **caractérisé en ce que** sur le tronçon rigide (4) est fixée une gaine de réception qui comporte une zone de support formant le support (18).

6. Endoscope selon la revendication 5, **caractérisé en ce que** la gaine de réception comporte une zone de fixation (19) à laquelle la gaine d'immobilisation (23) est directement reliée.

7. Endoscope selon l'une quelconque des revendications précédentes, **caractérisé en ce que** le tronçon rigide (4) est la zone d'extrémité distale (4) de la tige (3) de l'endoscope.

8. Endoscope selon la revendication 7, **caractérisé en ce que** la tige (3) de l'endoscope comporte entre la poignée (2) et le tronçon pliable (5) une partie principale (6) rigide qui est reliée au tronçon pliable (5), la partie principale (6) comportant un deuxième support, qui s'étend dans le sens longitudinal de la tige (3) de l'endoscope et sur lequel repose l'extrémité proximale du tube extérieur (11) flexible, une deuxième gaine d'immobilisation étant reliée à la partie principale (6) et s'engageant au-dessus de l'extrémité proximale du tube extérieur (11), de telle sorte que, en vue de l'immobilisation, celle-ci est bloquée entre la deuxième gaine d'immobilisation et le deuxième support.
